# EUROPÄISCHE PATENTSCHRIFT

(11) **EP 1 192 153 B1**
(45) Veröffentlichungstag und Bekanntmachung des Hinweises auf die Patenterteilung: **03.09.2003**
(21) Anmeldenummer: 00942012.6
(22) Anmeldetag: 05.06.2000
(51) Int. Cl.: C07D 471/04

(54) **VERFAHREN ZUR ENANTIOMERENTRENNUNG VON CIS-8-BENZYL-7,9-DIOXO-2,8-DIAZABICYCLO 4.3.0]NONAN**
METHOD FOR THE ENANTIOMER SEPARATION OF CIS-8-BENZYL-7,9-DIOXO-2,8-DIAZABICYCLO 4.3.0]NONANE
PROCEDE DE SEPARATION D'ENANTIOMERES CONTENUS DANS CIS-8-BENZYLE-7,9-DIOXO-2,8-DIAZABICYCLO 4.3.0]NONANE

(30) Priorität: 16.06.1999 DE 19927412
(43) Veröffentlichungstag der Anmeldung: 03.04.2002
(73) Patentinhaber: Bayer Aktiengesellschaft, 51368 Leverkusen (DE)
(72) Erfinder: FEY, Peter, D-42111 Wuppertal (DE)
(86) Internationale Anmeldenummer: EP0005116
(87) Internationale Veröffentlichungsnummer: WO00076996

(56) Entgegenhaltungen:
- EP-A- 0 550 903
- WO-A-93/13087
- US-A- 3 682 925

## Beschreibung

Die vorliegende Erfindung betrifft ein Verfahren zur Enantiomerentrennung von cis-8-Benzyl-7,9-dioxo-2,8-diazabicyclo[4.3.0]nonan (im folgenden auch als cis-6-Benzyl-5,7-dioxooctahydropyrrolo[3,4-b]pyridin oder Dioxopyrrolopiperidin bezeichnet). In einem weiteren Aspekt betrifft die Erfindung ein Verfahren zur Herstellung von (1S,6R)- und (1R,6S)-8-Benzyl-7,9-dioxo-2,8-diazabicyclo-[4.3.0]nonan unter Verwendung des zuvorgenannten Verfahrens. Weiterhin betrifft die Erfindung die (-)-2,3:4,6-Di-O-isopropyliden-2-keto-L-gulonsäuresalze von (1S,6R)- und (1R,6S)-8-Benzyl-7,9-dioxo-2,8-diazabicyclo[4.3.0]nonan sowie ein Verfahren zu deren Herstellung. Ferner betrifft die Erfindung ein Verfahren zur Enantiomerenanreicherung von (1S,6R)-8-Benzyl-7,9-dioxo-2,8-diazabicyclo[4.3.0]-nonan.

(1S,6R)-8-Benzyl-7,9-dioxo-2,8-diazabicyclo[4.3.0]nonan der Formel (Ia) 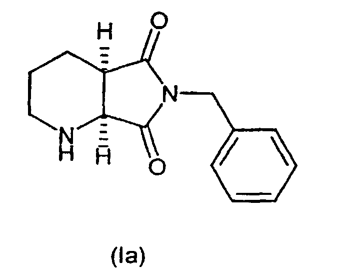 stellt ein wertvolles Zwischenprodukt für die Herstellung des (S,S)-2,8-diazabicyclo[4.3.0]nonan dar: 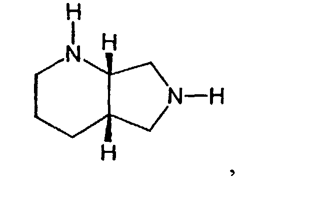 in das es durch Reduktion der Carbonylgruppen und Debenzylierung in an sich bekannter Weise (EP-A-0350733) überführt werden kann. Das (S,S)-2,8-Diazabicyclo[4.3.0]nonan wird seinerseits für die Herstellung des Antibiotikums Moxifloxacin (INN) verwendet: 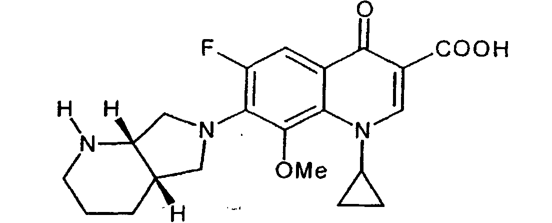 1-Cyclopropyl-7-([S,S]-2,8-diazabicyclo[4.3.0]non-8-yl)-6-fluor-1,4-dihydro-8-methoxy-4-oxo-3-chinoloncarbonsäure (Moxifloxacin) (EP-A-0350733).

Auch das Enantiomere 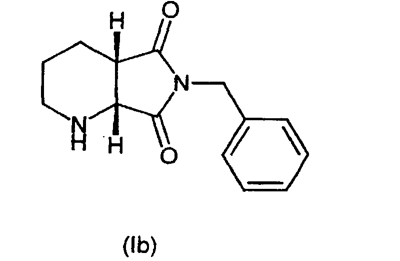 stellt ein wertvolles Zwischenprodukt für die Herstellung des (R,R)-2,8-diazabicyclo[4.3.0]nonans dar: 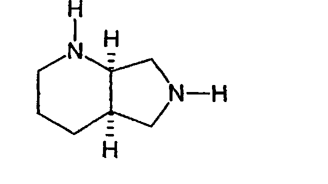 das seinerseits für die Herstellung sehr wirksamer antibakterieller Mittel verwendet werden kann (z.B. Interscience Conference on Antimicrobial Agents and Chemotherapy (ICAAC), 1996, Abstr. No. F-001).

Es bestand daher der Wunsch nach einem kostengünstigen Verfahren zur Herstellung des (1S,6R)-8-Benzyl-7,9-dioxo-2,8-diazabicyclo[4.3.0]nonans und des (1R,6S)-8-Benzyl-7,9-dioxo-2,8-diazabicyclo[4.3.0]nonans aus dem racemischen cis-8-Benzyl-7,9-dioxo-2,8-diazabicyclo[4.3.0]nonan, das durch Kernhydrierung von Pyridin-2,3-dicarbonsäure-N-benzylimid wie in der EP-A-0 350 733 (Beispiel K) beschrieben, erhalten wird.

Aus der EP-A-0 550 903 ist ein Verfahren zur Racematspaltung des cis-8-Benzyl-7,9-dioxo-2,8-diazabicyclo[4.3.0]nonans unter Verwendung von Weinsäure bekannt (Beispiel A, Methode IV sowie Beispiel B, Methode II a)). Die dort beschriebenen Verfahren erfordern für die Herstellung z.B. des (1S,6R)-Enantiomers das mehrmalige Umkristallisieren der diastereomeren D-(-)-Weinsäuresalze bzw. die Umsetzung mit L-(+)-Weinsäure sowie nachfolgende Umsetzung der freigesetzten Mutterlauge mit D-(-)-Weinsäure und Umkristallisation. Die erhaltenen Enantiomerenüberschüsse sind mit 93,8 % ee für das (1R,6S)-Enantiomer und 96,6 % ee für das (1S,6R)-Enantiomer unzureichend. Das Verfahren leidet unter dem Nachteil, dass die Bereitstellung des gewünschten Enantiomers in ausreichender Reinheit langwierige Operationen erfordert. Das Verfahren ist daher für die Produktion des (1S,6R)-8-Benzyl-7,9-dioxo-2,8-diazabicyclo[4.3.0]nonans in industriellem Maßstab ungeeignet.

Die Aufgabe der vorliegenden Erfindung bestand daher darin ein Verfahren zur Enantiomerenanreicherung von cis-8-Benzyl-7,9-dioxo-2,8-diazabicyclo[4.3.0]nonan bereitzustellen, mit dem das (1S,6R)-8-Benzyl-7,9-dioxo-2,8-diazabicyclo[4.3.0]-nonan oder das (1R,6S)-8-Benzyl-7,9-dioxo-2,8-diazabicyclo[4.3.0]nonan kostengünstig in großen Mengen und der geforderten Reinheit bereitgestellt werden kann.

Der Erfinder unternahm daher intensive Untersuchungen, die Probleme der Racematspaltung unter Verwendung von cis-8-Benzyl-7,9-dioxo-2,8-diazabicyclo[4.3.0]-nonan zu lösen. Dabei untersuchte er verschiedene im Stand der Technik bekannte chirale Säuren auf ihre Eignung, die vorstehend beschriebenen Aufgaben zu lösen. Dabei zeigte sich, dass zahlreiche im Stand der Technik zur Racematspaltung von Aminen erwähnte chirale Säuren für die Lösung der Aufgabenstellung ungeeignet waren. Schließlich gelang es völlig überraschend ein wirtschaftliches Verfahren zur Enantiomerenanreicherung von cis-8-Benzyl-7,9-dioxo-2,8-diazabicyclo[4.3.0]nonan zu finden, das (-)-2,3:4,6-Di-O-isopropyliden-2-keto-L-gulonsäure verwendet. (-)-2,3:4,6-Di-O-isopropyliden-2-keto-L-gulonsäure ist zur Racematspaltung von Aminen an sich bekannt (US-A-3,682,925). Es zeigte sich jedoch überraschend, dass bei Verwendung dieser speziellen chiralen Säuren das (1S,6R)-8-Benzyl-7,9-dioxo-2,8-diazabicyclo[4.3.0]nonan und das (1R,6S)-8-Benzyl-7,9-dioxo-2,8-diazabicyclo[4.3.0]nonan in besonders einfacher Weise in wenigen Schritten mit hoher Reinheit kostengünstig erhalten werden kann.

Gegenstand der Erfindung ist somit ein Verfahren zur Enantiomerenanreicherung von cis-8-Benzyl-7,9-dioxo-2,8-diazabicyclo[4.3.0]nonan, dass die Umsetzung von Enantiomerengemischen des cis-8-Benzyl-7,9-dioxo-2,8-diazabicyclo[4.3.0]nonan der Formel (I) 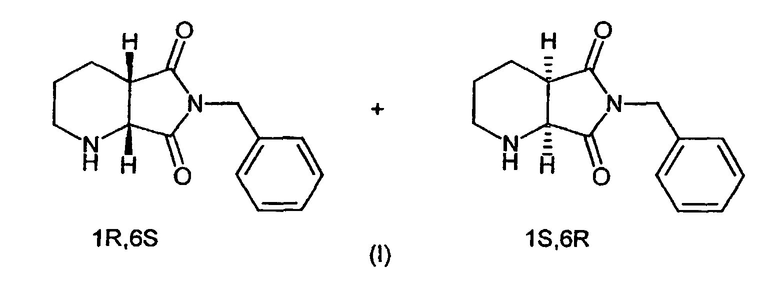 mit (-)-2,3:4,6-Di-O-isopropyliden-2-keto-L-gulonsäure oder deren Hydraten der Formel (II) umfaßt:

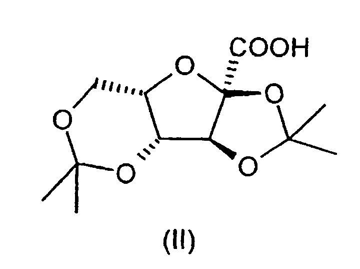

Enantiomerenanreicherung meint hier, dass das molare Verhältnis des einen Enantiomeren zum anderen erhöht wird bis hin zur vollständigen Enantiomerentrennung. Das Enantiomerengemisch des cis-8-Benzyl-7,9-dioxo-2,8-diazabicyclo-[4.3.0]nonan schließt beliebige Mischungen der Enantiomere ein und ist nicht auf das Racemat (1:1-Gemisch) beschränkt. So können auch Gemische des (1S,6R)- und (1R,6S)-8-Benzyl-7,9-dioxo-2,8-diazabicyclo[4.3.0]nonans im molaren Verhältnis von z.B. 80:20 zu 20:80 eingesetzt werden.

Die Umsetzung des Enantiomerengemisches mit der (-)-2,3:4,6-Di-O-isopropyliden-2-keto-L-gulonsäure erfolgt in Lösung oder Suspension. Als Lösungsmittel können organische Lösungsmittel oder Gemische davon mit bis zu 20 Gew.-% Wasser eingesetzt werden. Diese organischen Lösungsmittel werden bevorzugt ausgewählt aus der Gruppe, die besteht aus Alkoholen (z.B. Methanol, Ethanol, n-Propanol, Isopropanol, Isobutanol, n-Butanol und sek.-Butanol), Ketonen (z.B. Aceton, Methylethylketon, Methylisobutylketon) sowie Ether (wie z.B. Tetrahydrofuran, Dimethoxyethan), Acetonitril, Essigester, Toluol, Dimethylformamid. Auch Gemische der organischen Lösungsmittel können verwendet werden. Bevorzugt sind Ketone, besonders bevorzugt Methylethylketon.

Das Zusammengeben des Enantiomerengemisches und der (-)-2,3:4,6-Di-O-isopropyliden-2-keto-L-gulonsäure kann an sich in beliebiger Weise erfolgen. Zweckmäßig erfolgt das Zusammengeben jedoch, in dem man z.B. die Säure im jeweiligen Lösungsmittel gelöst oder suspendiert zu einer Lösung oder Suspension des Enantiomerengemisches gibt. Die Zugabe erfolgt bei Raumtemperatur (20°C) oder erhöhter Temperatur von bis etwa 110°C. Die Umsetzung des Enantiomerengemisches und der (-)-2,3:4,6-Di-O-isopropyliden-2-keto-L-gulonsäure erfolgt bevorzugt bei erhöhter Temperatur von 60 bis 110°C, bevorzugt bei der Siedetemperatur des jeweiligen Lösungmittels. Die Umsetzung kann gegebenfalls auch unter Druck erfolgen. Die Umsetzung der Amine mit den Säuren erfolgt an sich spontan. Es kann jedoch zweckmäßig sein, die Umsetzung für einen längeren Zeitraum von etwa 10 Minuten bis zu mehreren Stunden (z.B. maximal 20 Stunden, bevorzugt etwa bis zu 2 Stunden) durchzuführen, da durch Umlösungsprozesse der beiden Diastereomeren-Salze eine Anreicherung eines der beiden Diastereomeren-Salze unter den gegebenen Bedingungen (Temperatur, Lösungsmittel) erfolgen kann.

Das molare Verhältnis des racemischen Amins und der Säure beträgt zweckmäßig 1:0,4 bis 1:1. Bevorzugt beträgt das molare Verhältnis des racemischen Amins und der Säure 1:0,4 bis 1:0,6 und besonders bevorzugt beträgt das molare Verhältnis etwa 1:0,5. Der Grund dafür, warum man bevorzugt mit einem molaren Unterschuß der Säure arbeitet besteht darin, dass das (1R,6S)-8-Benzyl-7,9-dioxo-2,8-diazabicyclo[4.3.0]nonan-(-)-2,3:4,6-Di-O-isopropyliden-2-keto-L-gulonsäuresalz bevorzugt ausfällt. Das (1S,6R)-8-Benzyl-7,9-dioxo-2,8-diazabicyclo[4.3.0]nonan-enantiomer bzw. das entsprechende Gulonsäuresalz bleiben in Lösung. Da das gewünschte Produkt das freie (1S,6R)-Amin ist, ist eine vollständige Umsetzung desselben zum Gulonsäuresalz zur Abtrennung vom anderen Enantiomer nicht erforderlich und wirtschaftlich auch nicht wünschenswert.

Nach der Umsetzung wird das Reaktionsgemisch auf die Filtrationstemperatur von beispielsweise 5°C bis 40°C abgekühlt. Die Filtrationstemperatur wird so gewählt, dass gewünschte Diastereomeren-Salz in maximaler Ausbeute und Reinheit anfällt.

Bei dem Verfahren der Erfindung fällt in der Regel, wie oben beschrieben, zunächst das (1R,6S)-8-Benzyl-7,9-dioxo-2,8-diazabicyclo[4.3.0]nonan-(-)-2,3:4,6-Di-O-isopropyliden-2-keto-L-gulonsäuresalz aus, das durch Filtration in hoher Reinheit isoliert wird. Durch einmaliges Umkristallisieren kann das Salz praktisch diastereomerenrein erhalten werden. Aus dem Salz kann durch Umsetzung mit Basen in an sich bekannter Weise das freie Amin gewonnen werden.

Das nichtumgesetzte (1S,6R)-8-Benzyl-7,9-dioxo-2,8-diazabicyclo[4.3.0]nonan sowie das (-)-2,3:4,6-Di-O-isopropyliden-2-keto-L-gulonsäuresalz davon bleiben in der Regel in Lösung. Aus der Lösung kann das Salz durch weiteres Abkühlen auskristallisiert werden, oder das freie Amin direkt durch Umsetzung mit Basen erhalten und durch Aufkonzentrieren isoliert werden.

Mit dem oben beschriebenen Verfahren zur Enantiomerentrennung können allgemein Enantiomerengemische der Formel (I), die einen molaren Überschuß eines der beiden Enantiomere enthalten, hergestellt und in geeigneter Weise weiterverwendet werden.

Das Verfahren der Erfindung eignet sich wie oben beschrieben zur Herstellung von (1S,6R)-8-Benzyl-7,9-dioxo-2,8-diazabicyclo[4.3.0]nonan der Formel (Ia) 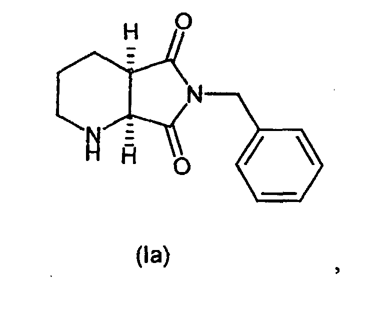 und zur Herstellung von (1R,6S)-8-Benzyl-7,9-dioxo-2,8-diazabicyclo[4.3.0]nonan der Formel (Ib)

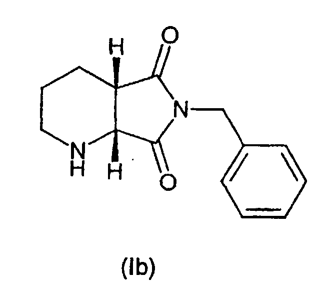

Bei dem Verfahren der Erfindung erfolgt bevorzugt die Freisetzung der Amine aus den (-)-2,3:4,6-Di-O-isopropyliden-2-keto-L-gulonsäuresalzen mit Basen. Die Freisetzung der Amine erfolgt bevorzugt mit einer Base, die ausgewählt wird aus der Gruppe die aus Natriumhydrogencarbonat und Ammoniak besteht.

Gegenstand der Erfindung sind somit auch (1S,6R)-8-Benzyl-7,9-dioxo-2,8-diazabicyclo[4.3.0]nonan-(-)-2,3:4,6-Di-O-isopropyliden-2-keto-L-gulonsäuresalz der Formel 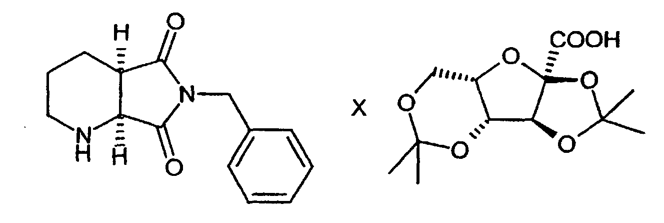 sowie (1R,6S)-8-Benzyl-7,9-dioxo-2,8-diazabicyclo[4.3.0]nonan-(-)-2,3:4,6-Di-Oisopropyliden-2-keto-L-gulonsäuresalz der Formel 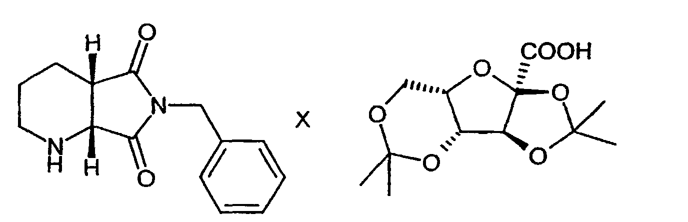 die als neue Zwischenprodukte bei dem Verfahren der Erfindung anfallen und aus denen die entsprechenden Amine in einfacher Weise isoliert werden können. Auch Gemische der (1R,6S)- und (1S,6R)-8-Benzyl-7,9-dioxo-2,8-diazabicyclo[4.3.0]-nonan-(-)-2,3:4,6-Di-O-isopropyliden-2-keto-L-gulonsäuresalze können nach dem Verfahren der Erfindung erhalten werden und ggf. weiterverarbeitet werden.

Gegenstand der Erfindung ist weiterhin ein Verfahren zur Enantiomerenanreicherung von (1S,6R)-8-Benzyl-7,9-dioxo-2,8-diazabicyclo[4.3.0]nonan der Formel (Ia) 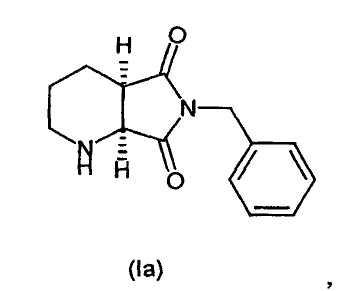 das die Umsetzung einer Lösung eines Enantiomerengemisches des cis-8-Benzyl-7,9-dioxo-2,8-diazabicyclo[4.3.0]nonans der Formel (I) in dem ein molarer Überschuß von (1S,6R)-8-Benzyl-7,9-dioxo-2,8-diazabicyclo[4.3.0]nonan vorliegt, wobei das Enantiomerengemisch des cis-8-Benzyl-7,9-dioxo-2,8-diazabicyclo[4.3.0]nonans ganz oder teilweise als Salz einer chiralen Säure vorliegen kann, mit Salzsäure umfaßt.

Bei dem Verfahren zur Enantiomerentrennung von cis-8-Benzyl-7,9-dioxo-2,8-diazabicyclo[4.3.0]nonan, dass die Umsetzung von Enantiomerengemischen des cis-8-Benzyl-7,9-dioxo-2,8-diazabicyclo[4.3.0]nonan der Formel (I) mit (-)-2,3:4,6-Di-O-isopropyliden-2-keto-L-gulonsäure oder deren Hydraten der Formel (II) umfaßt, fällt wie oben beschrieben ein Filtrat an, in dem ein Überschuß des (1S,6R)-8-Benzyl-7,9-dioxo-2,8-diazabicyclo[4.3.0]nonan vorliegt, das je nach Verhältnis der eingesetzten (-)-2,3:4,6-Di-O-isopropyliden-2-keto-L-gulonsäure zum Amin, ganz oder teilweise als Salz der (-)-2,3:4,6-Di-O-isopropyliden-2-keto-L-gulonsäure vorliegen kann. Es wurde nun völlig überraschend gefunden, dass wenn man die so hinsichtlich des (1S,6R)-Enantiomeren angereicherte Lösung mit Salzsäure versetzt, praktisch racemisches cis-8-Benzyl-7,9-dioxo-2,8-diazabicyclo[4.3.0]nonan-Hydrochlorid ausfällt, wodurch in der Lösung praktisch enantiomerenreines (1S,6R)-8-Benzyl-7,9-dioxo-2,8-diazabicyclo[4.3.0]nonan bzw. dessen Hydrochlorid verbleibt. Offenbar besitzt das Racemat des Hydrochlorids eine stärkere Neigung zur Kristallisation als das Hydrochlorid des enantiomerenreinen (1S,6R)-8-Benzyl-7,9-dioxo-2,8-diazabicyclo[4.3.0]nonans, was zur weiteren Anreicherung des (1S,6R)-8-Benzyl-7,9-dioxo-2,8-diazabicyclo[4.3.0]nonans ausgenutzt werden kann. Prinzipiell kann dieses Verfahren auch bei Lösungen eingesetzt werden, die aus der Umsetzung anderer chiraler Säuren, wie beispielsweise Weinsäure, mit dem racemischen cis-8-Benzyl-7,9-dioxo-2,8-diazabicyclo[4.3.0]nonan erhalten werden, und in denen ein Überschuß bezüglich eines der Enantiomeren vorliegt.

Bei dem vorstehend beschriebenen Verfahren erfolgt die Freisetzung des (1S,6R)-8-Benzyl-7,9-dioxo-2,8-diazabicyclo[4.3.0]nonan aus seinem Hydrochlorid mit Basen, wobei die Base bevorzugt ausgewählt wird aus der Gruppe die aus Natriumhydrogencarbonat und Ammoniak besteht.

Die folgenden Schema zeigt beispielhaft das Verfahren zur Enantiomerenanreicherung der Erfindung:

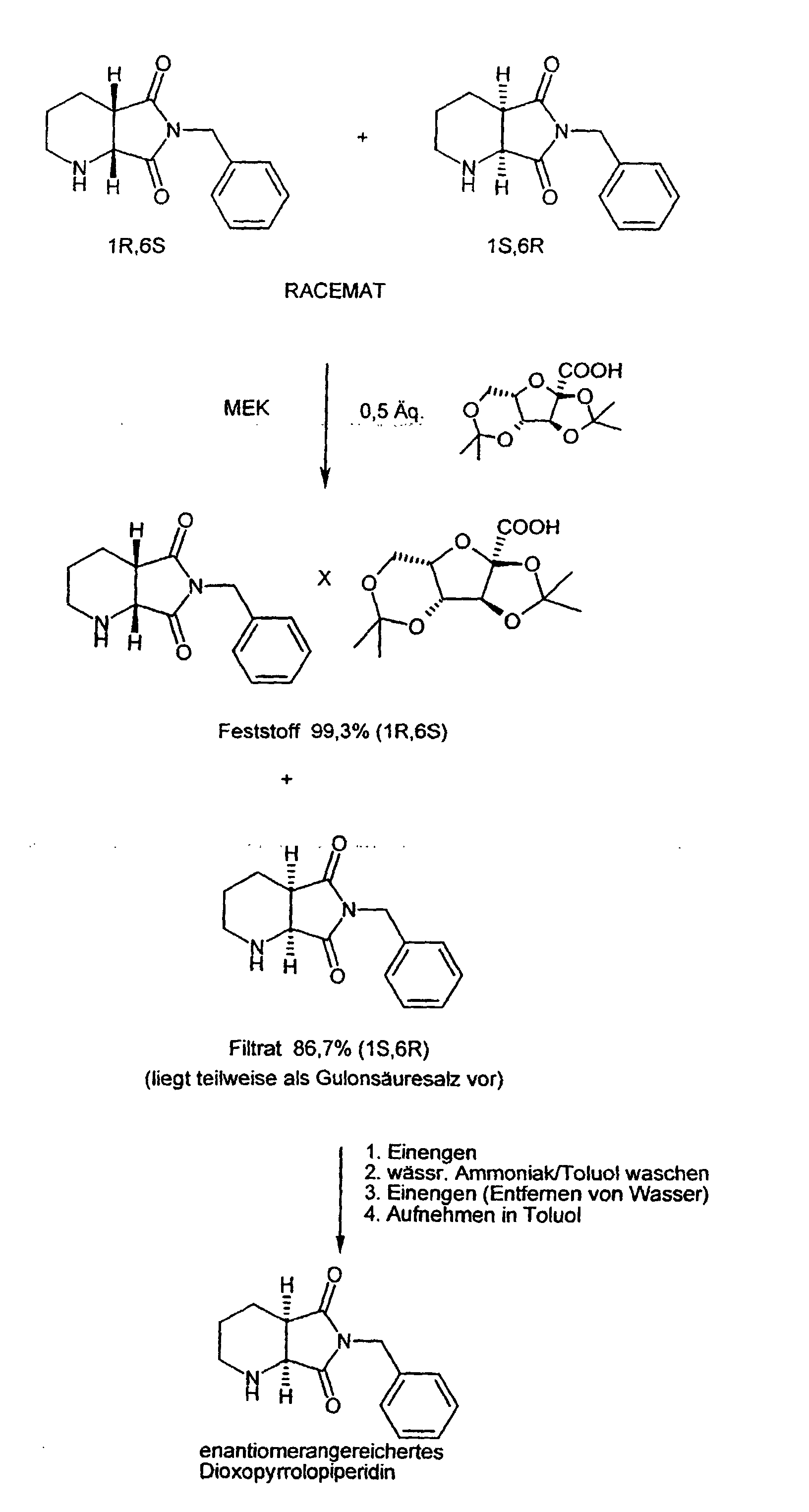

Das folgende Schema zeigt beispielhaft das Verfahren zur Enantiomerenanreicherung von (1S,6R)-8-Benzyl-7,9-dioxo-2,8-diazabicyclo[4.3.0]nonan durch Umsetzung eines Enantiomerengemisches, in dem ein molarer Überschuß von (1S,6R)-8-Benzyl-7,9-dioxo-2,8-diazabicyclo[4.3.0]nonan vorliegt, mit Salzsäure. Dabei wird das nach dem obigen Reaktionsschema erhaltene enantiomer angereicherte Filtrat mit Salzsäure umgesetzt:

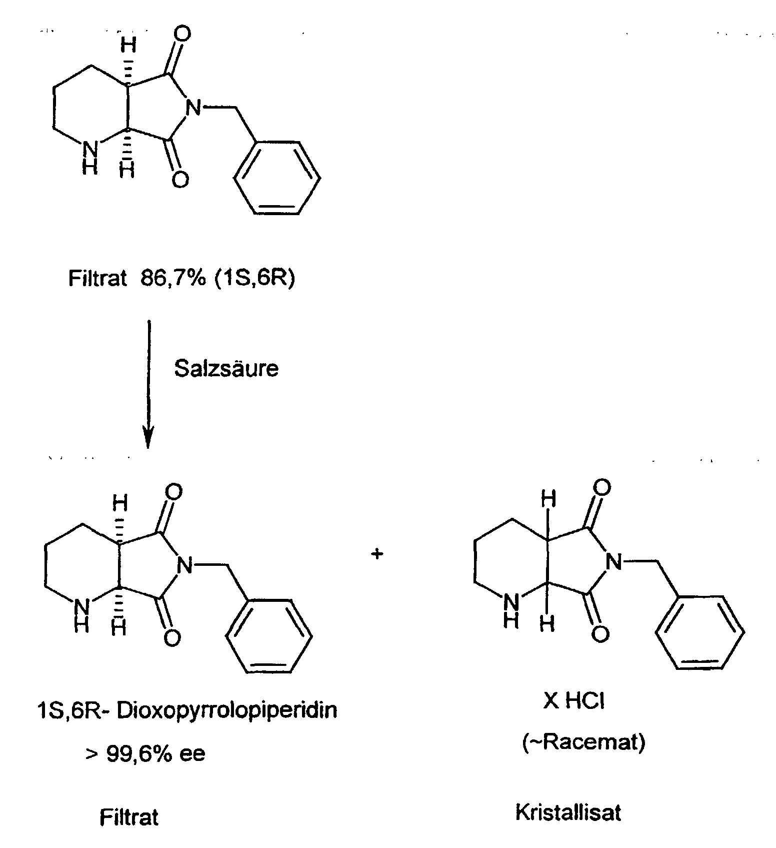

Das so angereicherte (1S,6R)-8-Benzyl-7,9-dioxo-2,8-diazabicyclo[4.3.0]nonan ((1S,6R)-Dioxopyrrolopiperidin) kann ohne weitere Aufarbeitung in die nachfolgende Reduktion mit Lithiumaluminiumhydrid eingesetzt werden:

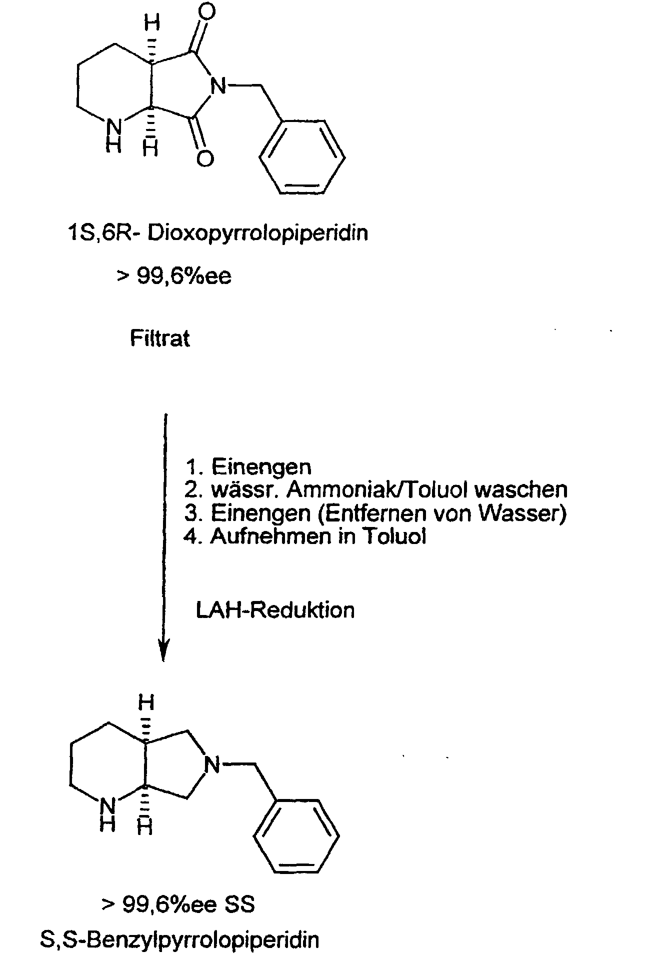

### Beispiele:

### Beispiel 1

### Racematspaltung von (1S,6R)- und (1R,6S)-8-Benzyl-7,9-dioxo-2,8-diazabicyclo-[4.3.0]nonan

### Dosiervariante A

2,0 kg (8,19 mol) cis-8-Benzyl-7,9-dioxo-2,8-diazabicyclo[4.3.0]nonan werden in 4,5 L (Liter) Methylethylketon suspendiert und auf Rückflußtemperatur (83°C) erhitzt. 1,2kg (-)-DAKS x H₂O [(-)-2,3:4,6-Di-O-isopropyliden-2-keto-L-gulonsäure-Monohydrat] und 1g Impfkristalle (1R,6S)-8-Benzyl-7,9-dioxo-2,8-diazabicyclo-[4.3.0]nonan-(-)-2,3:4,6-Di-O-isopropyliden-2-keto-L-gulonsäuresalz werden in 4,0 L Methylethylketon (MEK) suspendiert. Es wird ca. ¼ der Suspension zur siedenden cis-8-Benzyl-7,9-dioxo-2,8-diazabicyclo[4.3.0]nonan-Lösung gepumpt, wobei die Kristallisation einsetzt. Es wird 30Min. bei dieser Temperatur gerührt. Die restliche Suspension wind zugegeben, die Suspensionsvorlage und Pumpe mit 500ml Methylethylketon nachgespült und 1h bei Rückfluß nachgerührt. Es wird auf Raumtemperatur gekühlt, das Kristallisat wird abgesaugt und mit 1,2 L MEK gewaschen. Die Kristalle werden über Nacht bei 70°C im Vakuum getrocknet.

Ausbeute: 2037,1 g (1R,6S)-8-Benzyl-7,9-dioxo-2,8-diazabicyclo[4.3.0]nonan-(-)-2,3:4,6-Di-O-isopropyliden-2-keto-L-gulonsäuresalz, 86 % Enantiomerenüberschuß (ee).

Die vereinigten Filtrate werden im Vakuum zu einem Öl, das langsam kristallisiert, eingeengt.

Ausbeute: 1196 g (1S,6R)-8-Benzyl-7,9-dioxo-2,8-diazabicyclo[4.3.0]nonan (Rohprodukt, das teilweise als (-)-2,3:4,6-Di-O-isopropyliden-2-keto-L-gulonsäuresalz vorliegt). 146,2 g diescs Rohprodukts werden in 440 ml Toluol gelöst und mit 32 ml Wasser versetzt.

Es werden 5,1 ml Ammoniaklösung (25 %ig) zugegeben, auf 40°C erwärmt, die Phasen getrennt und die organische Phase im Vakuum bei 50°C eingeengt.
- Ausbeute:: 133,7 g (1S,6R)-8-Benzyl-7,9-dioxo-2,8-diazabicyclo[4.3.0]nonan 83,4 % ee

### Beispiel 2

### Racematspaltung von (1S,6R)- und (1R,6S)-8-Benzyl-7,9-dioxo-2,8-diazabicyclo[4.3.0]nonan

### Dosiervariante B

50 g (205 mmol) cis-8-Benzyl-7,9-dioxo-2,8-diazabicyclo[4.3.0]nonan werden in 111 mL Methylethylketon gelöst (trübe Lösung) und innerhalb 2 h zu einer siedenden Lösung von 29,9 g DAKS-Monohydrat in 114 mL Methylethylketon getropft. Die entstandene Suspension wird 10 min bei Siedetemperatur gerührt, auf Raumtemperatur abgekühlt und 2 h bei 20°C gerührt. Der Feststoff wird abgesaugt, mit 30 ml Methylethylketon gewaschen und im Vakuum bei 70°C getrocknet.
- Ausbeute:: 48,4 g (1R,6S)-8-Benzyl-7,9-dioxo-2,8-diazabicyclo[4.3.0]nonan-(-)-2,3:4,6-Di-O-isopropyliden-2-keto-L-gulonsäuresalz, 92,2 % ee.

Die vereinigten Filtrate werden eingeengt, in 50 mL Toluol und 6,3 mL Wasser gelöst und mit ca 1 mL 25 %iger Ammoniaklösung auf pH 10 eingestellt. Bei 40°C werden die Phasen getrennt, die organische Phase wird eingeengt und im Vakuum getrocknet.
- Ausbeute:: 25,8 g (1S,6R)-8-Benzyl-7,9-dioxo-2,8-diazabicyclo[4.3.0]nonan 84,8 % ee

### Beispiel 3

### Racematspaltung von (1S,6R)- und (1R,6S)-8-Benzyl-7,9-dioxo-2,8-diazabicyclo-[4.3.0]nonan

### Dosiervariante C

200 g (819 mmol) cis-8-Benzyl-7,9-dioxo-2,8-diazabicyclo[4.3.0]nonan und 120 g (411 mmol) DAKS-Monohydrat werden in 900 mL Methylethylketon suspendiert und zum Sieden erhitzt. Es wird 10 min bei Siedetemperatur gerührt, auf Raumtemperatur abgekühlt und 2 h bei 20°C gerührt. Der Feststoff wird abgesaugt , mit 120 ml Methylethylketon gewaschen und im Vakuum bei 70°C getrocknet.
- Ausbeute:: 216,9 g (1R,6S)-8-Benzyl-7,9-dioxo-2,8-diazabicyclo[4.3.0]nonan-(-)- 2,3:4,6-Di-O-isopropyliden-2-keto-L-gulonsäuresalz, 74,6 % ee

Filtrat: (1S,6R)-8-Benzyl-7,9-dioxo-2,8-diazabicyclo[4.3.0]nonan (Rohprodukt, das teilweise als (-)-2,3:4,6-Di-O-isopropyliden-2-keto-L-gulonsäuresalz vorliegt) 84,6 % ee.

### Beispiel 4

50 g (205 mmol) cis-8-Benzyl-7,9-dioxo-2,8-diazabicyclo[4.3.0]nonan werden in 111 mL Methylethylketon gelöst (trübe Lösung) und innerhalb 2 h zu einer siedenden Lösung von 29,9g DAKS-Monohydrat in 139 mL Methylethylketon getropft. Die entstandene Suspension wird 10 min bei Siedetemperatur gerührt, auf Raumtemperatur abgekühlt und 2 h bei 20°C gerührt. Der Feststoff wird abgesaugt, mit 80 ml Methylethylketon gewaschen und im Vakuum bei 70°C getrocknet.
- Ausbeute:: 49,1 g (1R,6S)-8-Benzyl-7,9-dioxo-2,8-diazabicyclo[4.3.0]nonan-(-)- 2,3 :4,6-Di-O-isopropyliden-2-keto-L-gulonsäuresalz.

Die vereinigten Filtrate werden mit 9,7 mL 37 %iger Salzsäure versetzt und 1h bei Raumtemperatur gerührt. Der entstandene Feststoff wird abgesaugt, mit 50 mL Methylethylketon gewaschen und im Vakuum bei 70°C getrocknet.

### Ausbeute: 4,9 g cis-8-Benzyl-7,9-dioxo-2,8-diazabicyclo[4.3.0]nonan-Hydrochlorid (Racemat)

Die vereinigten Filtrate werden mit 8,5 mL 25 %iger Ammoniaklösung auf pH 10 eingestellt, es werden 36 mL gesättigte Kochsalzlösung zugegeben, die organische Phasen wird getrennt, eingeengt und im Vakuum getrocknet.
- Ausbeute:: 22,0 g (1S,6R)-8-Benzyl-7,9-dioxo-2,8-diazabicyclo[4.3.0]nonan 99,6 % ee.

### Beispiel 5

### (1R,6S)-8-Benzyl-7,9-dioxo-2,8-diazabicyclo[4.3.0]nonan-(-)-2,3:4,6-Di-O-isopropyliden-2-keto-L-gulonsäuresalz.

2,0 kg (8,19 mol) cis-8-Benzyl-7,9-dioxo-2,8-diazabicyclo[4.3.0]nonan und 1,2 kg (-)-DAKS x H₂O [(-)-2,3:4,6-Di-O-isopropyliden-2-keto-L-gulonsäure-Monohydrat] werden in 11,4L Methylethylketon (MEK) und 0,57 L Wasser 30 min unter Rückfluß erhitzt. Es wird auf Raumtemperatur gekühlt, das Kristallisat wird abgesaugt und mit 3,2 L MEK gewaschen. Die Kristalle werden über Nacht bei 70°C im Vakuum getrocknet.
- Ausbeute:: 1753 g (1R,6S)-8-Benzyl-7,9-dioxo-2,8-diazabicyclo[4.3.0]nonan-(-)- 2,3:4,6-Di-O-isopropyliden-2-keto-L-gulonsäuresatz, 99,2 % Enantiomerenüberschuß (ee).

## Patentansprüche

1. Verfahren zur Enantiomerenanreicherung von cis-8-Benzyl-7,9-dioxo-2,8-diazabicyclo[4.3.0]nonan, das die Umsetzung von Enantiomerengemischen des cis-8-Benzyl-7,9-dioxo-2,8-diazabicyclo[4.3.0]nonan der Formel (I) 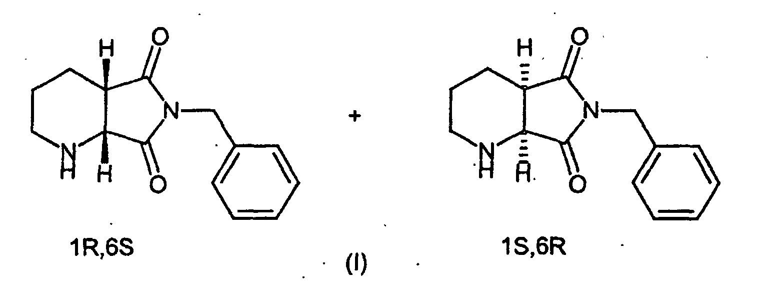 mit (-)-2,3:4,6-Di-O-isopropyliden-2-keto-L-gulonsäure oder deren Hydraten der Formel (II) 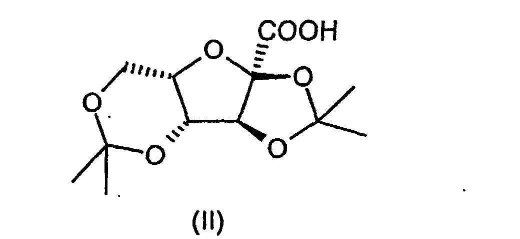 umfasst.

2. Verfahren zur Herstellung eines Enantiomerengemisches der Formel (I), enthaltend einen molaren Überschuss eines der beiden Enantiomere, wobei das Verfahren die Enantiomerentrennung nach Anspruch 1 umfasst.

3. Verfahren zur Herstellung von (1S,6R)-8-Benzyl-7,9-dioxo-2,8-diazabicyclo[4.3.0]nonan der Formel (Ia) 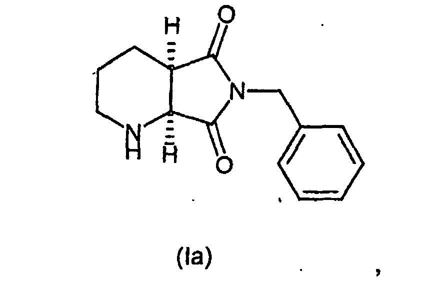 das das Verfahren nach Anspruch 1 umfasst.

4. Verfahren zur Herstellung von (1R,6S)-8-Benzyl-7,9-dioxo-2,8-diazabicyclo[4.3.0]nonan der Formel (Ib) 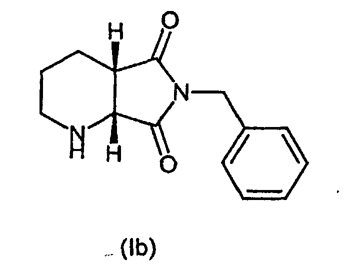 das das Verfahren nach Anspruch 1 umfasst.

5. Verfahren nach irgendeinem der Ansprüche 1 bis 4, das die Freisetzung der Amine aus den (-)-2,3:4,6-Di-O-isopropyliden-2-keto-L-gulonsäuresalzen mit Basen umfasst.

6. Verfahren nach Anspruch 5, worin die Base ausgewählt wird aus Natriumhydrogencarbonat und Ammoniak.

7. (1S,6R)-8-Benzyl-7,9-dioxo-2,8-diazabicyclo[4.3.0]nonan-(-)-2,3:4,6-Di-O-isopropyliden-2-keto-L-gulonsäuresalz der Formel

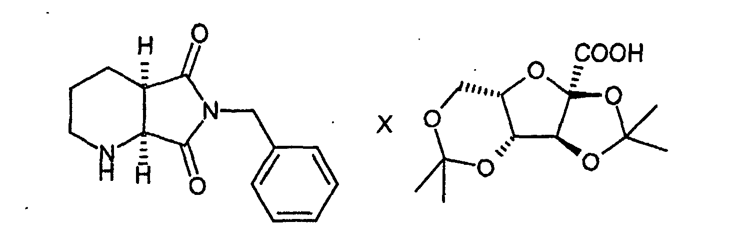

8. (1R,6S)-8-Benzyl-7,9-dioxo-2,8-diazabicyclo[4.3.0]nonan-(-)-2,3:4,6-Di-Oisopropyliden-2-keto-L-gulonsäuresalz der Formel

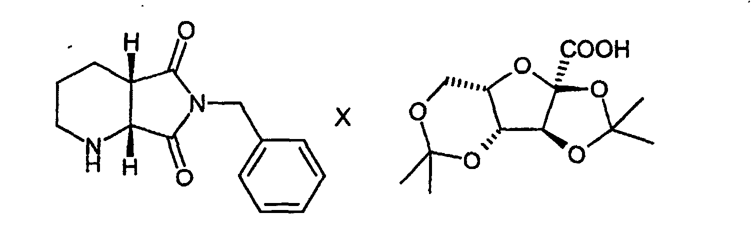

9. Gemische der Verbindungen nach Ansprüchen 7 und 8.

10. Verfahren zur Herstellung der Verbindungen nach Anspruch 8 oder 9, das das Verfahren nach Anspruch 1 umfasst.

11. Verfahren zur Enantiomerenanreicherung von (1S,6R)-8-Benzyl-7,9-dioxo-2,8-diazabicyclo[4,3.0]nonan der Formel (Ia) 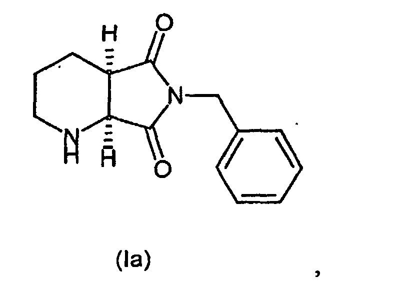 das die Umsetzung einer Lösung eines Enantiomerengemisches des cis-8-Benzyl-7,9-dioxo-2,8-diazabicyclo[4.3.0]nonans der Formel (I) mit Salzsäure umfasst, in dem ein molarer Überschuss von (1S,6R)-8-Benzyl-7,9-dioxo-2,8-diazabicyclo[4.3.0]nonan vorliegt, wobei das Enantiomerengemisch des cis-8-Benzyl-7,9-dioxo-2,8-diazabicyclo[4.3.0]nonans ganz oder teilweise als Salz einer chiralen Säure vorliegen kann.

12. Verfahren nach Anspruch 11, bei dem die Lösung des Enantiomerengemisches des cis-8-Benzyl-7,9-dioxo-2,8-diazabicyclo[4.3.0]nonans der Formel (I), in dem ein molarer Überschuss von (1S,6R)-8-Benzyl-7,9-dioxo-2,8-diazabicyclo[4.3.0]nonan vorliegt, aus der Umsetzung des Racemats des cis-8-Benzyl-7,9-dioxo-2,8-diazabicyclo[4.3.0]nonans mit (-)-2,3:4,6-Di-O-isopropyliden-2-keto-L-gulonsäure, wie in Anspruch 1 beschrieben, resultiert.

13. Verfahren nach Anspruch 11 oder 12, das die Freisetzung des (IS,6R)-8-Benzyl-7,9-dioxo-2,8-diazabicyclo[4.3.0]nonan aus seinem Hydrochlorid mit Basen umfasst.

14. Verfahren nach Anspruch 13, worin die Base ausgewählt wird aus Natriumhydrogencarbonat und Ammoniak.

15. (1S,6R)-8-Benzyl-7,9-dioxo-2,8-diazabicyclo[4.3.0]nonan-Hydrochlorid der Formel

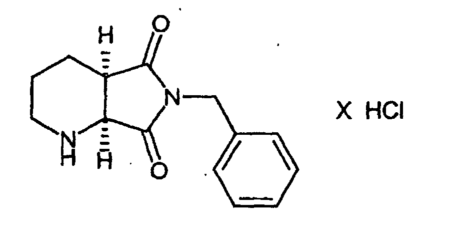

16. (1R,6S)-8-Benzyl-7,9-dioxo-2,8-diazabicyclo[4.3.0]nonan-Hydrochlorid der Formel

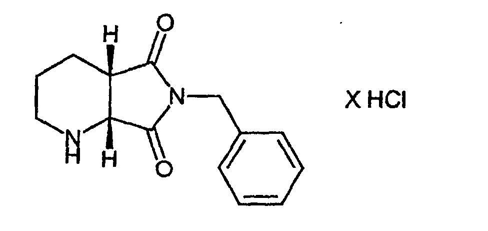

17. Gemische der Verbindungen nach Ansprüchen 15 und 16.

18. Verfahren zur Enantiomerenanreicherung aus cis-8-Benzyl-7,9-dioxo-2,8-diazabicyclo[4.3.0]nonan, wonach man ein Enantiomerengemisch des cis-8-Benzyl-7,9-dioxo-2,8-diazabicyclo[4.3.0]nonans der Formeln 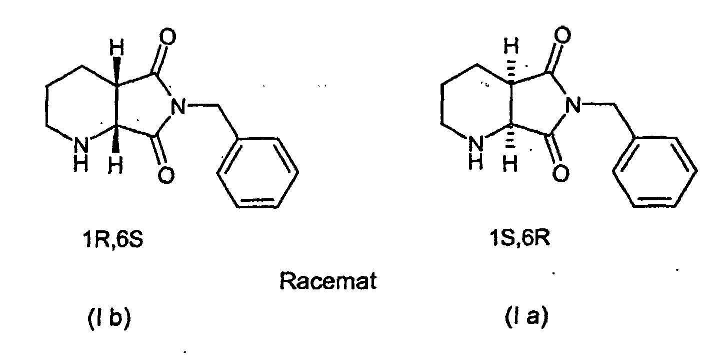 mit (-)-2,3:4,6-Di-O-isopropyliden-2-keto-L-gulonsäure der Formel 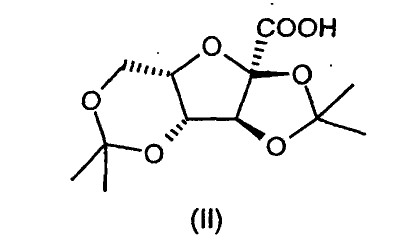 oder deren Hydraten umsetzt, feste ("a") und flüssige ("b") Phase dieser Umsetzung trennt,
• gegebenenfalls aus der festen Phase (a) das 1R,6S-Enantiomer I b freisetzt und gegebenenfalls isoliert und
• entweder aus der flüssigen Phase (b) das 1S,6R-Enantiomer I a durch Umsetzung mit Base freisetzt und gegebenenfalls isoliert
• oder die flüssige Phase (b) mit Salzsäure umsetzt,
• feste (c) und flüssige (d) Phase dieser Umsetzung trennt, wobei die feste Phase (c) dem Hydrochlorid des Racemats (I a + I b) und die flüssige Phase (d) dem 1S,6R-Enantiomer(I a)-Hydrochlorid entsprechen, und
• durch Umsetzung mit Base aus der flüssigen Phase (d) das 1S,6R-Enantiomer I a und/oder aus der festen Phase (c) das racemische freie Amin und gegebenenfalls isoliert.

## Claims

1. Process for enantiomer enrichment of cis-8-benzyl-7,9-dioxo-2,8-diazabicyclo[4.3.0]nonane, wherein the reaction of mixtures of enantiomers of cis-8-benzyl-7,9-dioxo-2,8-diazabicyclo[4.3.0]nonane of the formula (I) 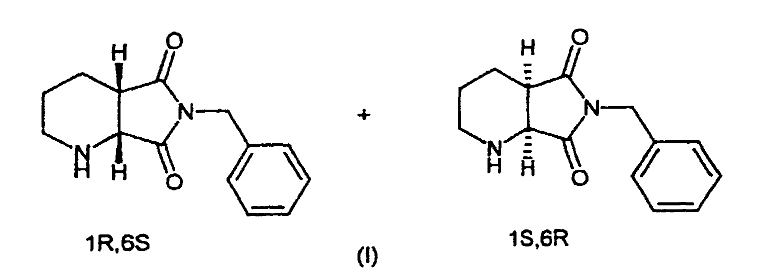 with (-)-2,3:4,6-di-O-isopropylidene-2-keto-L-gulonic acid or its hydrates of the formula (II) 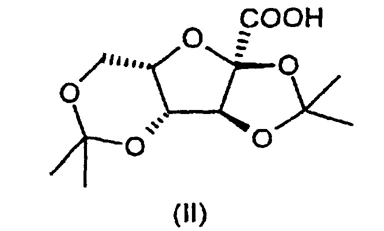 is comprised.

2. Process for the preparation of a mixture of enantiomers of the formula (I) that contains a molar excess of one of the two enantiomers, said process comprising the enantiomer separation according to Claim 1.

3. Process for the preparation of (1S,6R)-8-benzyl-7,9-dioxo-2,8-diazabicyclo[4.3.0]nonane of the formula (Ia) 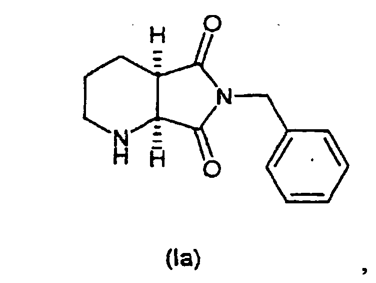 that comprises the process according to Claim 1.

4. Process for the preparation of (1S,6R)-8-benzyl-7,9-dioxo-2,8-diazabicyclo[4.3.0]nonane of the formula (Ib) 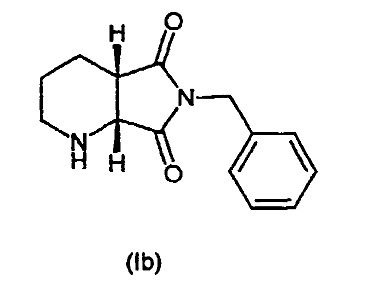 that comprises the process according to Claim 1.

5. Process according to any one of Claims 1 to 4, which comprises the release of the amines from the (-)-2,3:4,6-di-O-isopropylidene-2-keto-L-gulonic acid salts using bases.

6. Process according to Claim 5, in which the base is selected from sodium hydrogencarbonate and ammonia.

7. (1S,6R)-8-Benzyl-7,9-dioxo-2,8-diazabicyclo[4.3.0]nonane-(-)-2,3:4,6-di-O-isopropylidene-2-keto-L-gulonic acid salt of the formula

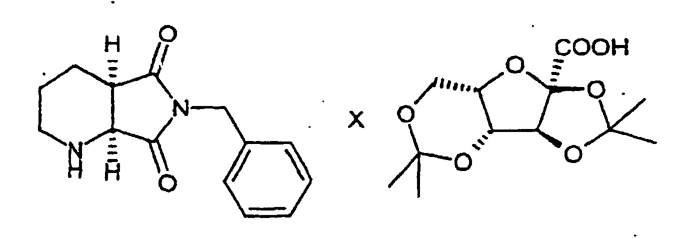

8. (1R,6S)-8-Benzyl-7,9-dioxo-2,8-diazabicyclo[4.3.0]nonane-(-)-2,3:4,6-di-O-isopropylidene-2-keto-L-gulonic acid salt of the formula

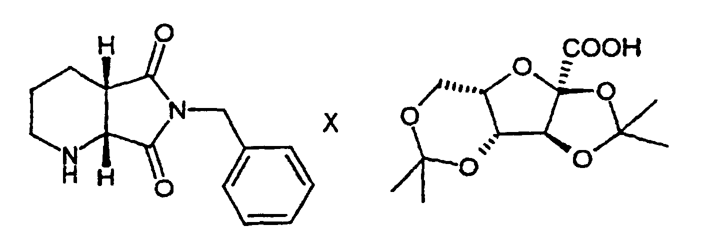

9. Mixtures of the compounds according to Claims 7 and 8.

10. Process for the preparation of the compounds according to Claim 8 or 9 which comprises the process according to Claim 1.

11. Process for the enrichment of enantiomers of (1S,6R)-8-benzyl-7,9-dioxo-2,8-diazabicyclo[4.3.0]nonane of the formula (Ia) 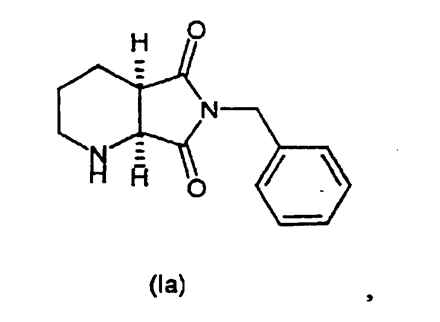 which comprises the reaction of a solution of a mixture of enantiomers of cis-8-benzyl-7,9-dioxo-2,8-diazabicyclo[4.3.0]nonane of the formula (I) in which a molar excess of (1S,6R)-8-benzyl-7,9-dioxo-2,8-diazabicyclo[4.3.0]nonane is present, it being possible for the mixture of enantiomers of cis-8-benzyl-7,9-dioxo-2,8-diazabicyclo[4.3.0]nonane to be present wholly or partially as a salt of a chiral acid, with hydrochloric acid.

12. Process according to Claim 11, in which the solution of the mixture of enantiomers of cis-8-benzyl-7,9-dioxo-2,8-diazabicyclo[4.3.0]nonane of the formula (I), in which a molar excess of (1S,6R)-8-benzyl-7,9-dioxo-2,8-diazabicyclo[4.3.0]nonane is present, results from the reaction of the racemate of cis-8-benzyl-7,9-dioxo-2,8-diazabicyclo[4.3.0]nonane with (-)-2,3:4,6-di-O-isopropylidene-2-keto-L-gulonic acid, as described in claim 1.

13. Process according to Claim 11 or 12, which comprises the release of (I S,6R)-8-benzyl-7,9-dioxo-2,8-diazabicyclo[4.3.0]nonane from its hydrochloride using bases.

14. Process according to Claim 13, in which the base is selected from sodium hydrogencarbonate and ammonia.

15. (1S,6R)-8-Benzyl-7,9-dioxo-2,8-diazabicyclo[4.3.0]nonane hydrochloride of the formula

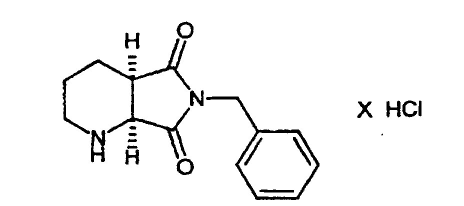

16. (1R,6S)-8-Benzyl-7,9-dioxo-2,8-diazabicyclo[4.3.0]nonane hydrochloride of the formula

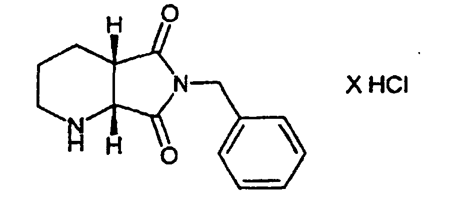

17. Mixtures of the compounds as claimed in claims 15 and 16.

18. Process for enantiomer enrichment from cis-8-benzyl-7,9-dioxo-2,8-diazabicyclo[4.3.0]nonane, wherein a mixture of enantiomers of cis-8-benzyl-7,9-dioxo-2,8-diazabicyclo[4.3.0]nonane of the formulae 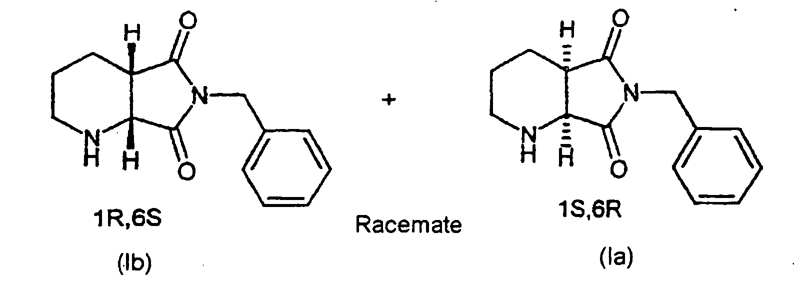 is reacted with (-)-2,3:4,6-di-O-isopropylidene-2-keto-L-gulonic acid of the formula 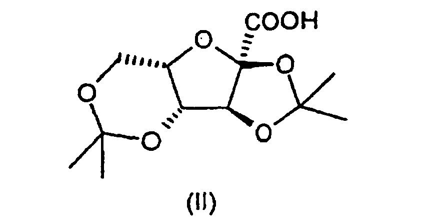 or its hydrates, solid ("a") and liquid ("b") phases of this reaction are separated,
• the 1R,6S-enantiomer I b is optionally released from the solid phase (a) and optionally isolated and
• either the 1S,6R-enantiomer 1 a is released from the liquid phase (b) by reaction with base and optionally isolated
• or the liquid phase (b) is reacted with hydrochloric acid,
• solid (c) and liquid (d) phases of this reaction are separated, the solid phase (c) corresponding to the hydrochloride of the racemate (I a + I b) and the liquid phase (d) corresponding to the 1S,6R-enantiomer I a hydrochloride, and
• the 1S,6R-enantiomer I a is released from the liquid phase (d) and/or the racemic free amine from the solid phase (c) by reaction with base and optionally isolated.

## Revendications

1. Procédé de concentration des énantiomères du cis-8-benzyl-7,9-dioxo-2,8-diazabicyclo[4.3.0]nonane, qui comprend la réaction de mélanges d'énantiomères du cis-8-benzyl-7,9-dioxo-2,8-diazabicyclo[4.3.0]nonane de formule (I) 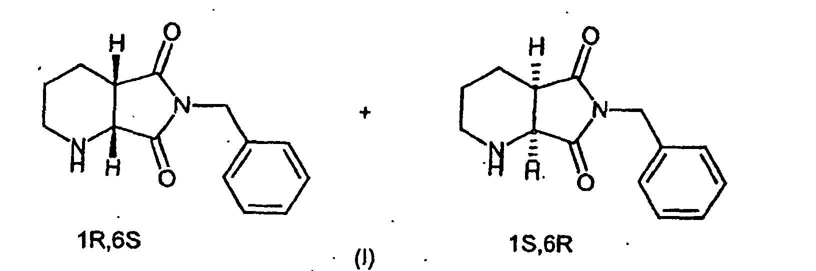 avec l'acide (-)-2,3:4,6-di-O-isopropylidène-2-céto-L-gulonique ou ses hydrates, de formule (II)

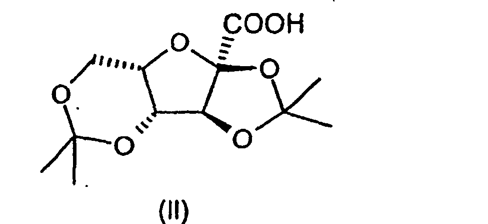

2. Procédé de production d'un mélange d'énantiomères de formule (I), contenant un excès molaire de l'un des deux énantiomères, procédé qui comprend la séparation des énantiomères selon la revendication 1.

3. Procédé de production du (1S,6R)-8-benzyl-7,9-dioxo-2,8-diazabicyclo[4.3.0]nonane de formule (Ia) 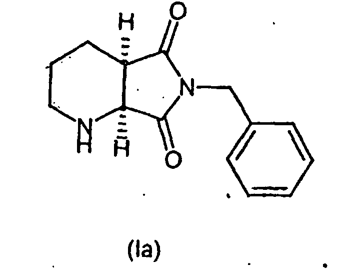 qui comprend le procédé suivant la revendication 1.

4. Procédé de production du (1R,6S)-8-benzyl-7,9-dioxo-2,8-diazabicyclo[4.3.0]nonane de formule (Ib) 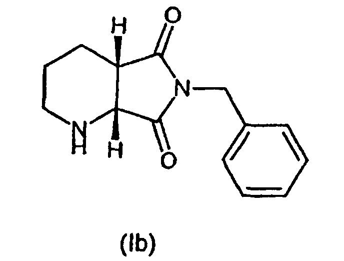 qui comprend le procédé suivant la revendication 1.

5. Procédé suivant l'une quelconque des revendications 1 à 4, qui comprend la libération des amines des sels de l'acide (-)-2,3:4,6-di-O-isopropylidène-2-céto-L-gulonique avec des bases.

6. Procédé suivant la revendication 5, dans lequel la base est choisie entre le bicarbonate de sodium et l'ammoniac.

7. Sel d'acide (1S,6R)-8-benzyl-7,9-dioxo-2,8-diazabicyclo[4.3.0]nonane-(-)-2,3:4,6-di-O-isopropylidène-2-céto-L-gulonique de formule

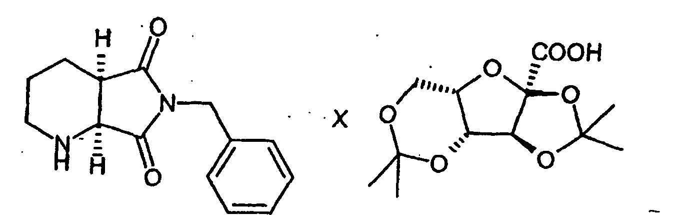

8. Sel d'acide (1R,6S)-8-benzyl-7,9-dioxo-2,8-diazabicyclo[4.3.0]nonane-(-)-2,3:4,6-di-O-isopropylidène-2-céto-L-gulonique de formule

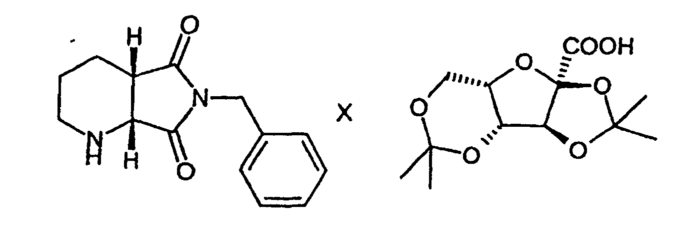

9. Mélanges des composés suivant les revendications 7 et 8.

10. Procédé de production des composés suivant la revendication 8 ou 9, qui comprend le procédé suivant la revendication 1.

11. Procédé de concentration des énantiomères du (1S,6R)-8-benzyl-7,9-dioxo-2,8-diazabicyclo[4.3.0]nonane de formule (Ia) 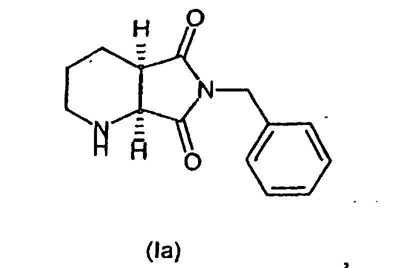 qui comprend la réaction d'une solution du mélange d'énantiomères du cis-8-benzyl-7,9-dioxo-2,8-diazabicyclo-[4.3.0]nonane de formule (I) avec l'acide chlorhydrique, dans lequel un excès molaire du (1S,6R)-8-benzyl-7,9-dioxo-2,8-diazabicyclo[4.3.0]nonane est présent, le mélange d'énantiomères du cis-8-benzyl-7,9-dioxo-2,8-diazabicyclo-[4.3.0]nonane pouvant être présent en totalité ou en partie sous forme d'un sel d'un acide chiral.

12. Procédé suivant la revendication 11, dans lequel la solution du mélange d'énantiomères du cis-8-benzyl-7,9-dioxo-2,8-diazabicyclo[4.3.0]nonane de formule (I), dans lequel un excès molaire du (1S,6R)-8-benzyl-7,9-dioxo-2,8-diazabicyclo[4.3.0]nonane est présent, résulte de la réaction du racémate du cis-8-benzyl-7,9-dioxo-2,8-diazabicyclo[4.3.0]nonane avec l'acide (-)-2,3:4,6-di-O-isopropylidène-2-céto-L-gulonique comme décrit dans la revendication 1.

13. Procédé suivant la revendication 11 ou 12, qui comprend la libération du (1S,6R)- 8-benzyl-7,9-dioxo-2,8-diazabicyclo[4.3.0]nonane de son chlorhydrate avec des bases.

14. Procédé suivant la revendication 13, dans lequel la base est choisie entre le bicarbonate de sodium et l'ammoniac.

15. Le chlorhydrate de (1S,6R)-8-benzyl-7,9-dioxo-2,8-diazabicyclo[4.3.0]nonane de formule

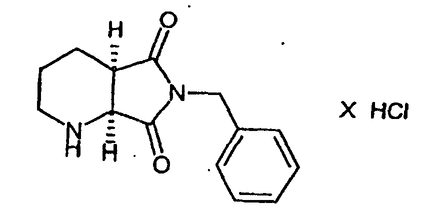

16. Le chlorhydrate de (1R,6S)-8-benzyl-7,9-dioxo-2,8-diazabicyclo[4.3.0]nonane de formule

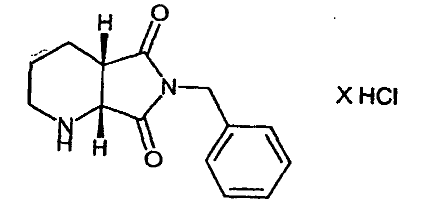

17. Mélanges des composés suivant les revendications 15 et 16.

18. Procédé de concentration des énantiomères de cis-8-benzyl-7,9-dioxo-2,8-diazabicyclo[4.3.0]nonane, dans lequel on fait réagir un mélange d'énantiomères du cis-8-benzyl-7,9-dioxo-2,8-diazabicyclo[4.3.0]nonane de formules 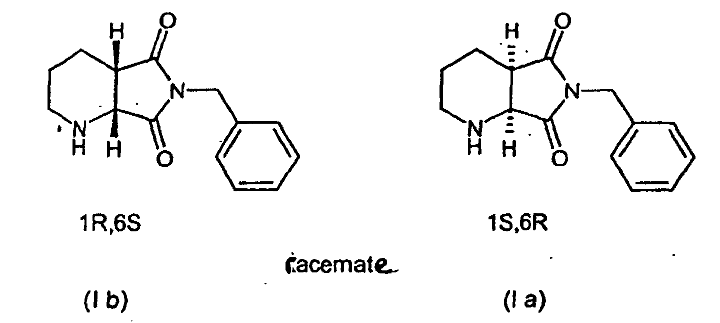 avec l'acide (-)2,3:9,6-di-O-isopropylidène-2-céto-L-gulonique de formule 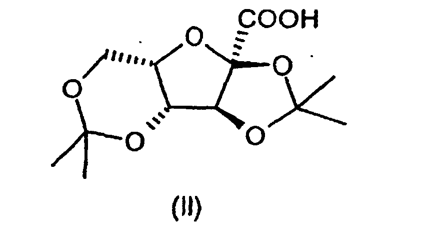 ou ses hydrates, on sépare une phase solide ("a") et une phase liquide ("b") de cette réaction,
• on libère éventuellement le 1R,6S-énantiomère Ib de la phase solide (a) et on l'isole, le cas échéant, et
• on libère le 1S,6R-énantiomè Ia de la phase liquide (b) par réaction avec une base et on l'isole, le cas échéant,
• ou bien on fait réagir la phase liquide (b) avec l'acide chlorhydrique,
• on sépare la phase solide (c) et la phase liquide (d) de cette réaction, la phase solide (c) correspondant au chlorhydrate du racémate (Ia + Ib) et la phase liquide (d) correspondant au chlorhydrate du 1S,6R-énantiomère (Ia), et
• on libère par réaction avec une base le 1S,6R-énantiomère Ia de la phase liquide (d) et/ou l'amine libre racémique de la phase solide (c) et on l'isole, le cas échéant.
